# EUROPEAN PATENT APPLICATION

(11) **EP 4 209 723 A1**
(43) Date of publication of application: **12.07.2023**
(21) Application number: 21759328.4
(22) Date of filing: 28.07.2021
(51) Int. Cl.: F24F 8/15, F24F 8/22, F24F 8/26, F24F 8/30, F24F 11/58, F24F 110/10, F24F 110/20, F24F 110/66, F24F 110/70, F24F 110/72, A61L 9/20, A61L 9/22

(54) **DEVICE FOR GENERATING HYDROXYL RADICALS**

(30) Priority: 04.09.2020 EP 20382789
(71) Applicant: Ohmnia Global Technologies, S.L., 08173 Sant Cugat del Vallés Barcelona (ES)
(72) Inventor: BECERRIL RUIZ, Jose Luis, 08750 Molins de Rei (Barcelona) (ES)
(74) Representative: Torras Toll, Jorge
(86) International application number: PCT/ES2021/070576
(87) International publication number: WO 2022/049314

(57) **Abstract**

This invention refers to a device for generating hydroxyl radicals comprising: a) at least one container suitable for containing a substance from which hydroxyl radicals are generated; b) at least one conversion unit suitable for generating hydroxyl radicals from that substance; c)_a fluid conduction system that allows air to be recirculated inside the device in such a way that part of the air with a mixture of radicals circulates several times before going outside and d) a base comprising a power supply, a fan, an air inlet and outlet duct from the device, means for electrically supplying the various components of the device and a controller; in which the air inlet and air outlet duct include a mixing vessel before the air outlet of the device, characterised in that these vessel and conversion unit are independent of each other and can be independently removed from the base.

## Description

The present invention relates to a device for the sterilisation of indoor environments through the generation of hydroxyl radicals (•OH), ions and other radicals. With the device of the present invention it is possible to very effectively eliminate pollutants of biological origin such as bacteria and viruses, as well as pollutants present in the air, such as carbon monoxide, sulphur dioxide or nitrogen dioxide.

With the development of industrialisation and the increase of the world population, air pollution has increased and every day new pathologies related to agents of biological origin, such as viruses and bacteria appear, which makes pollution in closed spaces more serious. On the other hand, the increase in population density favours contagion between individuals, increasing the risk in indoor environments, which makes it necessary to establish distance measures and continuous cleaning and sterilisation processes for surfaces exposed to possible contamination. All these factors increase the need for continuous treatment of air and surfaces that may have a contaminating load.

The inhalation of bacterial or fungal spores or viral particles in suspension is the cause of a wide range of diseases. The air inhaled by people, contaminated with these microorganisms, and which may also contain volatile organic compounds, can be the cause of several diseases and can lead to systemic infections and allergies.

Nowadays, many technologies using different methods for the removal or adsorption of these pollutants exist. One example is the use of filtration means. The efficiency of a filtration system is also limited by the size of the contaminating particles, as filters are not adapted to particles of very different sizes. Filters also trap biological matter, which is a point of contagion if replacement or maintenance is not carried out correctly. In addition, filter systems have no effect on surfaces, their range of action is limited and they have a high cost.

On the other hand, there are ion generators that increase the ion charge in the air by generating and releasing mainly negatively-charged ions into the air. One application would be, for example, PCI technology (Plasma Cluster Ionisation), which consists in the generation of plasma ions, releasing positive and negative ions. The charged particles then adhere to surfaces in the room, where they can cause allergies or asthma problems. In addition, they can produce dangerous levels of ozone and do not produce hydroxyl radicals.

Photocatalysis oxidation (PCO) technology consists of irradiation with ultraviolet light of surfaces coated with titanium dioxide (TiO₂) to generate free radicals. With this, all the air has to be passed through the device. Most studies concur it is effective only on surfaces coated with TiO₂, which means that it only acts on pollutants that are in contact with these surfaces or very close to them, and therefore its effectiveness is limited. Although some of said devices can release hydroxyl radicals, tests have concluded that they produce very low quantities with a high dependence on relative ambient humidity.

In conclusion, most state-of-the-art systems are deficient, either because they are only effective for specific pollutants, because they treat only part of the indoor air but not the surfaces, because they generate by-products that can be harmful to health in general, or because they cannot be in constant operation or used when people are present. In addition, they all have high energy consumption. It is therefore necessary to develop a device that can overcome the abovementioned shortcomings.

The inventors of the present invention have developed a device for generating hydroxyl radicals (•OH) which improves hygiene conditions in indoor spaces by acting on the air and on soft and porous solid surfaces, such as fabrics, present in those spaces and thus improving the quality of life of humans in particular, and other living beings in general, with very low energy consumption.

The device, for generating hydroxyl radicals of the present invention also allows the generation of other radicals, such as the hydroperoxyl radical (•HO₂), and of negative and/or positive ions.

The radicals •OH and •HO₂ are formed naturally when ultraviolet sunlight reacts with water vapour (H₂O) and ozone (O₃) in the lower layers of the atmosphere. The hydroxyl radical (•OH), a molecule formed by a hydrogen atom and an oxygen atom with an unpaired electron, is one of the most reactive gases in the atmosphere. It acts as a "detergent" in the air, breaking down other gases and killing viruses and bacteria. In particular, •OH is the main controller on methane concentrations, a powerful greenhouse gas, that is the second largest contributor to global warming after carbon dioxide.
- OH plays a very important role in the natural purification of pollutants as it is the main cleaning agent of the atmosphere, removing several pollutants such as carbon monoxide, sulphur dioxide and nitrogen dioxide. •OH radicals have been shown not only to be completely harmless to the health of plants, animals and humans, but also to act effectively in the air, on surfaces of solid materials, as well as in textiles and other porous materials.

There are devices for generating hydroxyl radicals in the prior art from the ozonolysis of hydrogen peroxide and terpenes such as d-limonene, but the generation of hydroxyl radicals produced by such equipment is inefficient. In these devices a simple mixingis performed and do not take the amount of time into account to make a complete mixture of both substances, reactant and oxidant. In addition, they do not take into account either the need to generate the turbulence necessary for the mixture to be done completely. This causes small concentrations of unreacted substances, both ozone and reactant, which are subsequently released into the air without reacting. It's very important not to release ozone into the air as high concentrations could be produced and generate by-products derived from the reaction of this gas with the pollutants present in the air, apart from causing allergic reactions and health conditions in the people who are in continuous contact with small amounts of ozone. The toxicity of ozone concentrations even in small quantities has been well documented.

One way to increase the efficiency of the process is to try to consume as much part of the ozone in the reaction with the reactant base and decompose the reactant base completely, in such a way as to ensure maximum production of hydroxyl groups, which allows an optimal, fast and effective effect in the elimination of contaminants of both microbiological and chemical origin.

Examples of said hydroxyl radical generation devices are those that are disclosed in patent applications WO 2012/059726 and WO 2020/087225. The components of these devices are basically a deposit with reactant (terpene or hydrogen peroxide), which is released by evaporation or with ultrasound within the same reactant container, and an ozone and ion generator. Ozone mixes with the reactant base and hydroxyl radicals are generated. These systems, although they generate hydroxyl radicals, can be inefficient, given that the path of said hydroxyl radicals to the outlet is very short, the mixture leaves directly without generating a suitablemixing and part of the ozone and the reactant is released into the air without reacting. This generates potential contaminants and produces less amount of hydroxyl, which leads to a decrease in the ability to removal of contaminants.

H2O2 initiates the decomposition of O3 by electron transfer; alternatively, the reaction can be thought of as the activation of H2O2 by ozone, which is known as peroxone. Among the possible mixtures of oxidant agents, the combination of hydrogen peroxide and ozone is undoubtedly the most used. The process aims to combine the direct (and selective) oxidation of ozone with the rapid and not very selective reaction of • OH radicals. But the mixing process must be done repeatedly in order to fully take advantage of the synergistic effects of the oxidants generated, creating a complete reaction of the peroxide and ozone.

The present inventors have surprisingly discovered that the increase in the amount of hydroxyl radicals released does not depend on the release method of the reactant (vaporization, ultrasound, nebulization, among others) but of the system's ability to achieve a complete reaction of both substances. To achieve the complete reaction of the reactant with the ions of ozone, a device has been developed in which it is recirculated at least once or it performs at least one cycle of the mixture through the reactant base and through the unit generator of ozone and ions. This way, oxidants and hydroxyl radicals generated in the first round help to completely break down the reactant base and decompose redundant ozone and, consequently, more radicals and hydroxyl groups are generated. Also, this mix, before going into the air, is enhanced by using a mixing vessel, which generates turbulence.

Therefore, this present invention discloses a device for generating hydroxyl radicals comprising:
a) at least one container suitable for containing a substance from which said hydroxyl radicals are generated;
b) at least one conversion unit, suitable for generating said hydroxyl radicals from said substance; and
c) a base comprising a power supply, a fan, an air inlet and outlet duct of the device, means for electrically supplying the various components of the device and a controller; wherein the air inlet and outlet duct of the device comprises a mixing vessel before the air outlet of the device,
characterised in that said at least one container and at least one conversion unit are independent of each other and can be removed from the base unit, characterised in that it comprises at least an air recirculation air system, suitable to allow air with the mixture of hydroxyl radicals to recirculate at least once inside the device before going out.

The device of the present invention has the advantage that both the conversion unit and the container of the substance from which hydroxyl radicals are generated are independent of each other, i.e. they are not part of the same cartridge, and moreover they are removable, so that each one can be easily replaced, for example manually, retaining only the base. This allows for a reduction of the cost of the device, since it is not necessary to replace the container and the conversion unit at the same time. This advantage with respect to state-of-the-art devices is especially important since the substance from which hydroxyl radicals are generated is exhausted long before the conversion unit, so the device of the present invention has the advantage that the substance container can be replaced without having to replace the conversion unit at the same time.

In addition, the fact that both the conversion unit and the container can be disassembled and replaced independently of each other allows multiple combinations of the substance and the conversion unit to suit different applications of the device. Thus, the generation of hydroxyl radicals, ions and other radicals can be optimised according to various parameters, such as the charge or quantity required of each of them depending on the different types of contaminants or the location or size of the application. Therefore, the device of the present invention allows total adaptability to the specific conditions of each application.

As already detailed above, the device for generating hydroxyl radicals of the present invention has a base comprising an air inlet and outlet duct from the device.

Preferably, said air inlet and outlet duct comprises a first section, which conducts the air supplied by the fan; a second section connected to the first, which is narrower and to which the substance generated from the container arrives and mixes it with the substance generated from the conversor unit; a third section which returns the mixture of the fluids coming from the first two sections to the lower part of the first section; and a fourth section where the air from the first section is mixed with the mixture from the generator unit before leaving to the exterior of the device. Preferably, the fourth section comprises the mixing vessel of the air inlet and the outlet duct. It is in saidmixing vessel that the reaction that generates the hydroxyl radicals is completed before they are released from the device.

More preferably, the first and second section of the air inlet and outlet duct have respective diameters to create a Venturi effect to attract the air from the first and second section into the duct. Similarly, the third section has a diameter that will create a Venturi effect to draw the air coming from the first and second sections.

This air flow allows it to always go through the generator unit at least once, already mixed and charged with hydroxyl groups. Moreover, the air with mixture of substances from the generator unit is returned to the main channel in a wider section, expanding itself and easing the mixing process. This mixture will travel all the section to the upper part, where the air conduct is reduced, compressing the mixture and easing the combination of the different substances. Part of this air will flow through the generator unit again and another part will go to the mixing vessel, expanding once more and generating a turbulent flow due to the change in section size, facilitating even more the reaction of the generated agents, eliminating the possibility that there is any unmixed leftovers and maximizing radical generation.

In a preferred embodiment, the first section of the air inlet and outlet duct of the device comprise a primary air inlet channel, while the second section of the air inlet an outlet duct of the primary channel comprises a second air inlet channel from the first channel that returns the air with the mixture to the first channel. Said primary inlet channel is preferably located in the fan zone and said secondary air inlet channel is preferably located in the zone corresponding to the conversion unit and the container unit.

The first section has an opening that allows the access of part of the air to the second section. The second section has an outlet opening that allows the flow of air with the mixture into the third section.

The entrance opening to the second section and the air outlet from the third section to the main channel have a shape and diameter relation between them and the main channel in a way that from the opening located in the fan zone, a suction Venturi effect is produced, as well as a push effect from the opening located in the upper side of the conversion unit. That way, a recirculation of the air inside the second and third section is produced.

The person skilled in the art knows the diameter each section of the air duct should have for the Venturi effect and the impulsion to be created. A Venturi effect is a phenomenon by which the pressure of a moving fluid within a closed duct is decreased when its speed increases as it passes through an area of smaller section. When said speed increases very much, large differences in pressure can occur and then, if at that duct end another duct is introduced, then the fluid from that duct will be sucked in and mixed with the fluid circulating along the first duct.

Preferably, the container, also called the flask, is an open container containing a wick of a material selected from the list comprising cellulose, cotton, sponge or a mixture thereof. Said wick has the function of assisting in the evaporation of the substance or reagent from which the hydroxyl radicals are generated, so that it can be more easily transferred to the mixing vessel. Preferably, the substance from which hydroxyl radicals are generated will be a liquid selected from the group comprising terpenes such as limonene, myrcene, pinene, linalool or rosemary essential oil, hydrogen peroxide, water or a mixture of these. Preferably, said container does not include any electrical connections.

Preferably, the conversion unit is a plasma generator, capable of generating ions and small amounts of ozone, which reacts with the substance from which these hydroxyl radicals are generated, oxidising it and generating large amounts of them.

In another embodiment, said conversion unit can be a generator of ultraviolet light with a wavelength between 200 nm and 300 nm, generated by either lamps or lightemitting diodes (LEDs), which through photolysis are able to react with the substance from which hydroxyl radicals are generated without the use of any catalytic compound such as titanium dioxide (TiO₂) and without generating ozone.

Optionally, the device of the present invention may include a high frequency ultrasonic generator. By generating high frequency ultrasounds, usually in the range of 1.5 MHz to 2 MHz, the substance from which hydroxyl radicals are generated is dissociated. This method can be used both in air by acting on the gaseous phase of the substance from which hydroxyl radicals are generated, and by direct irradiation of the substance from which hydroxyl radicals are generated, in its liquid form. This produces cavitation bubbles that repeatedly grow and collapse. During the collapse, temperature and pressure increase exponentially causing the generation of large amounts of free radicals. In this case no ozone is generated. More preferably, the ultrasound generator may be installed in the mixing vessel or in the container. The ultrasound generator may be installed so that it only irradiates air with molecules of the substance from which hydroxyl radicals are generated, but can be installed immersed in the substance.

In a preferred embodiment, the conversion unit of the hydroxyl radical generator of this invention is an ozone (O₃) generator.

In a preferred embodiment, the conversion unit of the hydroxyl radical generator of this invention is a generator of positive and/or negative ions.

In the device from this invention, it is contemplated that more than one conversion unit may be used. It is also contemplated that when the conversion unit is an ultraviolet light generator, said conversion unit is installed in the container.

Preferably, the device from this invention also comprises sensors for the detection of volatile organic compounds, or sensors for particles in the air, or sensors for temperature and humidity, or sensors for the detection of carbon monoxide or carbon dioxide, among other. The purpose of these sensors is to determine the air quality.

Preferably, the device from this invention further comprises a data transmission module, for example, by means of Wi-Fi and/or Bluetooth, that allows an exchange of information with other devices. In this way, it is possible to control air quality remotely, as well as to control the device by means of mobile phones, tablets or any other device with Wi-Fi and/or Bluetooth connectivity.

Some embodiments of this invention are further detailed below with reference to the attached schematic figures in which:
Figure 1 shows a schematic view of an embodiment of the base and the conversion unit and the substance container unit from which hydroxyl radicals can be generated according to this invention.
Figure 2 shows a schematic view of another embodiment of the base, in which an ultraviolet light generator is used instead of an ultrasound generator.
Figure 3A and 3B show a schematic view of an embodiment of the device according this invention in which is the different sections the air flows through inside the device are shown.
Figure 4 shows a schematic view of an embodiment of the device according this invention in which it is shown how the conversion unit and the container of the substance are placed at the base of the device.
Figure 5 shows a schematic view of an embodiment of the device according this invention in which the way the conversion unit is docked to the base independently of the substance container unit is shown, therefore defining the corresponding place for each of the pieces inside of the device.
Figure 6 shows a graphwhere the hydrogen peroxide liberation to the air is compared between the present invention and a device from the state-of-art.

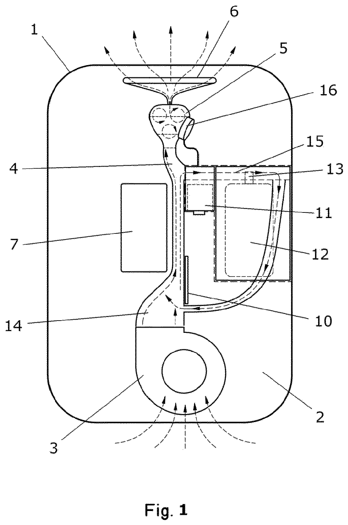

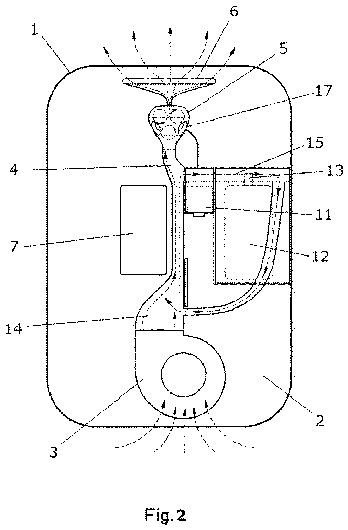

As shown in Figures 1 and 2, the device 1 from this invention comprises a base 2, in which there is a fan 3, an air duct 4 comprising a mixing vessel 5 near the air outlet 6 of the device 1. In addition, said base 2 comprises a controller 7 in the form of a base plate and a sensor 10. The air duct 4 has a wider first section and a narrower second section that gets even more narrower before the connection to the secondary channel.

In Figure 1, an ultrasound generator 16 that will be used in conjunction to the mixing vessel 5 to generate hydroxyl radicals is observed. In its place, in Figure 2 an ultraviolet light generator 17 with the same objective is observed, analogous to Figure 1.

As observed in Figure 1, air from the exterior gets inside the device from the fan zone 3, and is pushed to the primary channel 14, causing it to flow into the first and second section. This disposition gives the air acceleration and increases pressure, leading to the creation of a Venturi effect, that allows the total absorption of the air from the secondary channel 15 that contains the hydroxyl radicals generated by the conversion unit and the substance or the reactant. After that, the fluid from channel 15 that contains the hydroxyl radicals is recirculated through the tertiary section to end up once more in the first section, there it will go up and divide again. Thus, both the fluid from the primary channel 14 and the reabsorbed from secondary channel 15 are mixed and go into the mixing vessel 5, in which a turbulence is provoked, allowing an improvement in the mixing process and generation of hydroxyl radicals. After that, the air with the hydroxyl radicals is expulsed to the outside through the air outlet 6.

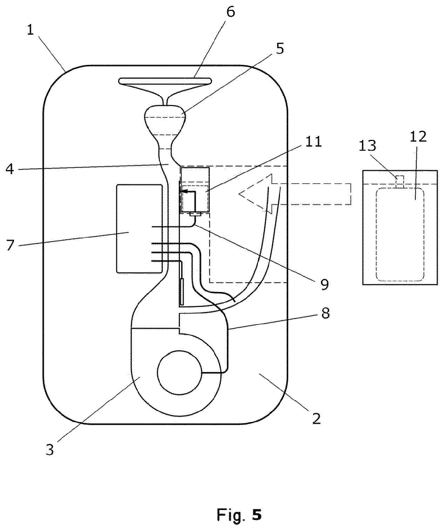

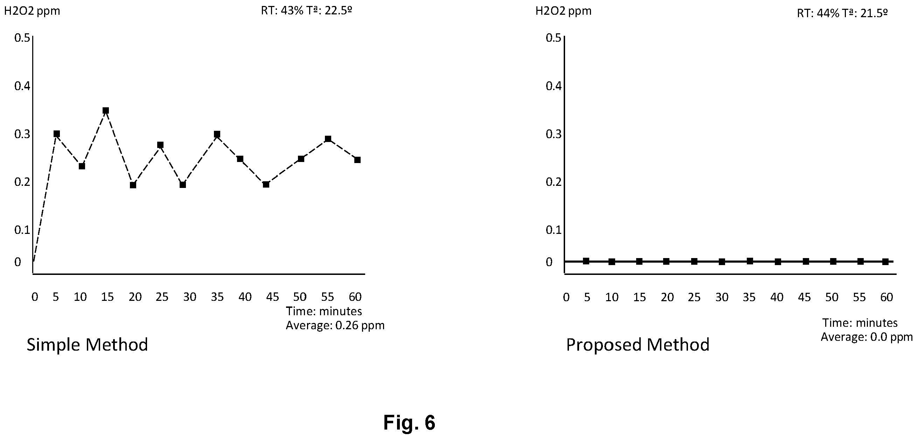

Optionally, the mixing vessel 5 can have a coating of parallel lines or in the form of a mesh made from a conductive metal material (not shown) and an ultrasonic generator 16 (Figure 5) or ultraviolet light 17 (Figure 6). Said generator improves the excitation and/or vibration of hydroxyl radical generator molecules, an effect that is enhanced by the metal cladding of the mixing vessel 5 and that allows for better mixing, thus improving the total efficiency of the process and minimizing the loss of reactives.

In order to cover a large area of interior space, the hydroxyl radical generator is able to generate a stable and sufficient amount of hydroxyl radicals through various reactions and exhibit superior effects in purifying and sterilising the air, therefore providing a higher quality of life and environment than is provided by currently available devices.

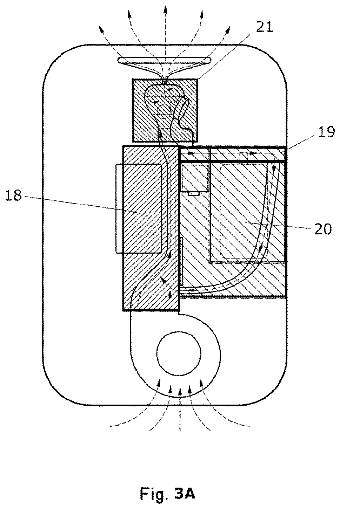

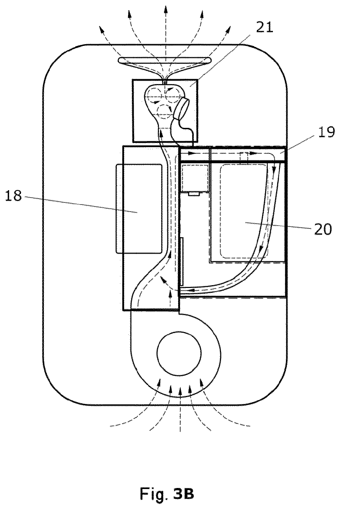

In Figures 3A and 3B, the different sections of air flow are observed: primary section 18, secondary section 19, tertiary section 20 and quaternary section 21.

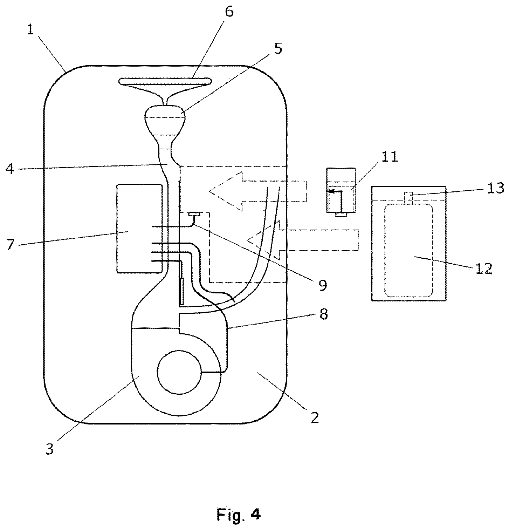

Figure 4 shows how the conversion unit 11 and the container unit 12 are located in the base 2. Arrows indicate how they both must be docked, independently from each other. The container that holds the reactant does not comprise any electrical connection, however, the conversion unit 11, be it in form of ionizer or in form or plasma generator (or others), comprises an electrical connection 9, the same way that the fan 3 has an electrical connection 8.

In Figure 5 it is observed that the conversion unit (ionized) 11 has been located in its place and it is also shown the container 12 suitable to contain a substance from which hydroxyl radicals can be generated. The arrow generate the position in which said container 12 in the base 2 is docked. Said container comprises a wick 13, to help evaporate the substance or reactant present in said container 12.

To cover a large area of interior space, the hydroxyl radical generating device of the present invention can generate a stable and sufficient amount of hydroxyl radicals through various reactions, and exhibits superior effects in air purification and sterilization, thus providing environmental and life quality superior to those provided by state-of-the-art devices.

### Examples

To optimize the amount of radicals released, the ability of the system to achieve a complete reaction of the elements is more important than the method of releasing the reactant (vaporization, ultrasound, nebulization, etc.). To achieve the complete reaction of all the elements, reactant with ions-ozone, it is necessary to mix several times by the reactant base and by the ozone and ion generator unit. In an optimal process, the reactant and oxidant mixture must pass through the generating unit at least twice so that the oxidants and radicals generated in the first round help decompose the ozone that had not reacted in it, and consequently decompose completely the reactant base and decompose the redundant ozone, thus generating a greater amount of radicals and hydroxyl groups. Furthermore, this generation is enhanced through the use of a mixing vessel 5 that generates turbulence and where elements such as ultrasound, ultraviolet or infrared light are added, applied to the air in that area where the mixture is located.

In the tests carried out, the difference in ozone concentration between the two devices mentioned in the patents named above is clearly observed, with mixing of ozone and simple reactant where the substances are mixed and released directly, and the difference for the purpose of this patent where they are performs repetitive mixing with bypass and turbulence generation.

There are no relevant differences in the amounts of vaporization / release of reactant as well as in the production of ozone and in both cases hydrogen peroxide is used as a base.

All tests are carried out and certified by independent laboratories according to International Standard EN 60335-2-69 (European Standard) or UL 867 (American Standard). The volume of the test space is of a similar size, about 25 m3 approx. and the same environmental conditions of 23° and 45% relative humidity +/- 10%.

### Example 1:

Regarding the method used in other embodiments, a simple outlet was carried out, with an evaporation method and without a mixing chamber (Simple Method). The equipment was in operation for 8h, where room concentrations of 1.6 x 10-6 (16 ppb) were reached.

In contrast, for the results referring to the object of this patent (proposed method), a cycle composed of more than one recirculation with the mixing vessel and during 24 hours of operation was carried out. The ozone concentration obtained in the room was always less than 0.01 X10-6 (1 ppb). This value coincides with the tolerance limit of the equipment, so it is considered as non-existent. It is important with this type of equipment to control ozone emissions, which over time can reach critical concentrations.

Hydrogen peroxide emission tests were also carried out in the air. The tests were carried out in the laboratory with a Dräger X-am^{®} 5100 device at a distance of 30 mm from the air outlet for both performances, obtaining the following results.

The proposed system of repetitive mixing with bypass and mixing vessel does not emit traces of hydrogen peroxide while the simple method in previous embodiments maintains an average emission of 0.26 ppm.

This lack of ozone and hydrogen peroxide release is explained because the entire generation has been consumed in the creation of hydroxyl radicals. This is also reflected in the results obtained in terms of reducing the microbiological load.

If we compare the results obtained in viral load reduction with both methods we get:

**Table 1: Antiviral effect of the different air circulation methods (previous/simple method and proposed method)**

| | Test Room | Volume m3 | Initial load Log. (10X) | Test Time | % Effectivity |
|---|---|---|---|---|---|
| Simple Method | Urn Box | 0,064 | 5 | 2h | 92% |
| Proposed Method | Room | 30 | 5 | <30 min | 100,00% |

The viruses used for the test are Mengovirus and Respiratory Syncytial Virus. The data in the first row are those provided by the developers of the previous method and were performed in an urn. The second row shows the certified data obtained with the proposed method and carried out in a room, demonstrating greater effectiveness in less time and in a much larger space.

Tests have also been carried out with bacteria (Escherichia coli) obtaining the following results:

**Table 2: Antiviral effect of the different air circulation methods (previous/simple method and proposed method)**

| | Test Room | Volume m3 | Initial load Log. (10X) | Test Time | % Effectivity |
|---|---|---|---|---|---|
| Simple Method | Room | 8 | 4 | 2h | 50% |
| Proposed Method | Room | 7,4 | 8 | 2h | 97,20% |

It is observed that even when the initial load is doubled, the proposed method is capable of reducing the bacterial load with much greater efficiency in the same time interval and at a similar volume.

In both viruses and bacteria the tests have been carried out repeatedly, obtaining the equivalent differences. The supplied amounts of Ozone and of reactant (hydrogen peroxide) are the same in all versions, both with the simple method and with the device of the present invention.

## Claims

1. A device for generating hydroxyl radicals comprising:
a) at least one container suitable for containing a substance from which said hydroxyl radicals are generated;
b) at least one conversion unit, suitable for generating said hydroxyl radicals from said substance;
c) at least one fluid conduction system that allows the recirculation of air within the device in such a way that at least part of the air with the radical mixture, circulates several times before going outside, and
d) a base comprising a power supply, a fan, an air inlet and outlet duct of the device, means for electrically supplying the various components of the device and a controller; wherein said air inlet and outlet duct of the device comprises a mixing vessel before the air outlet of the device,
**characterised in that** said at least one container and at least one conversion unit are independent of each other and can be removed from the base unit.

2. A device for generating hydroxyl radicals, according to claim 1, **characterised in that** the air inlet and outlet duct of the device comprises a first section, which conducts the air supplied by the fan; a second section connected to the first, which is narrower and where the substance generated from the container arrives; a third section connected to the second and first, which conducts the mixture of the fluids from the first two sections to the device outlet, and a fourth where the air is mixed and released to the outside.

3. A device for generating hydroxyl radicals, according to claim 2, **characterised in that** the first section comprises a primary air inlet channel, and the second section comprises a secondary air inlet channel.

4. A device for generating hydroxyl radicals, according to any one of the preceding claims, **characterised in that** the container is an open container comprising a wick of a material selected from the list comprising cellulose, cotton, sponge or a mixture thereof.

5. A device for generating hydroxyl radicals, according to any one of the preceding claims, **characterised in that** the substance from which hydroxyl radicals are generated is a liquid substance which is selected from the group comprising terpenes such as limonene, myrcene, pinene, linalool or rosemary essential oil, hydrogen peroxide, water or a mixture thereof.

6. A device for generating hydroxyl radicals, according to any one of the preceding claims, **characterised in that** the device also generates other radicals, such as hydroperoxyl radicals (-HO₂) and/or negative and/or positive ions and/or ozone (O₃).

7. A device for generating hydroxyl radicals, according to any one of the preceding claims, **characterised in that** its conversion unit is a plasma generator.

8. A device for generating hydroxyl radicals, according to any one of claims 1 to 6, **characterised in that** its conversion unit is an ultraviolet light generator.

9. A device for generating hydroxyl radicals, according to claim 8, **characterised in that** said ultraviolet light generator generates ultraviolet light with a wavelength between 200 nm and 300 nm.

10. A device for generating hydroxyl radicals, according to any one of claims 1 to 6, **characterised in that** said conversion unit is an ultrasound generator.

11. A device for generating hydroxyl radicals, according to claim 10, **characterised in that** said ultrasound generator generates at a frequency in the range of 1.5 MHz to 2 MHz.

12. A device for generating hydroxyl radicals, according to any one of claims 1 to 6, **characterised in that** said conversion unit is an ion generator.

13. A device for generating hydroxyl radicals, according to any one of claims 1 to 6, **characterised in that** said conversion unit is an ozone (O₃) generator.

14. A device for generating hydroxyl radicals, according to any one of the preceding claims, **characterised in that** it also comprises sensors for the detection of volatile organic compounds, or sensors for particles in the air, or temperature and humidity sensors, or sensors for the detection of carbon monoxide or carbon dioxide.

15. A device for generating hydroxyl radicals, according to any one of the preceding claims, **characterised in that** it also comprises a data transmission module for either Wi-Fi and/or Bluetooth transmissions.
